# EUROPEAN PATENT SPECIFICATION

(11) **EP 3 164 079 B1**
(45) Date of publication and mention of the grant of the patent: **28.12.2022**
(21) Application number: 15750456.4
(22) Date of filing: 01.07.2015
(51) Int. Cl.: A61B 8/08

(54) **LESION SIGNATURE TO CHARACTERIZE PATHOLOGY FOR SPECIFIC SUBJECT**
LÄSIONSSIGNATUR ZUR CHARAKTERISIERUNG DER PATHOLOGIE FÜR EINE BESTIMMTE PERSON
SIGNATURE DE LÉSION PERMETTANT DE CARACTÉRISER UNE PATHOLOGIE POUR UN SUJET SPÉCIFIQUE

(30) Priority: 02.07.2014 US 201462019926 P
(43) Date of publication of application: 10.05.2017
(73) Proprietor: Koninklijke Philips N.V., 5656 AG Eindhoven (NL)
(72) Inventor: JAGO, James Robertson, NL-5656 AE Eindhoven (NL); GAUTHIER, Thomas Patrice Jean Arsene, NL-5656 AE Eindhoven (NL); NG, Gary Cheng-How, NL-5656 AE Eindhoven (NL)
(74) Representative: Philips Intellectual Property & Standards
(86) International application number: PCT/IB2015/054945
(87) International publication number: WO 2016/001849

(56) References cited:
- US-A1- 2004 064 050
- US-A1- 2004 122 326
- US-A1- 2007 036 402
- US-A1- 2013 046 168
- US-A1- 2014 180 273
- MOMENAN R ET AL: "Characterization of tissue from ultrasound images", IEEE CONTROL SYSTEMS MAGAZINE, IEEE SEWRVICE CENTER, PISCATAWAY, NJ, US, vol. 8, no. 3, 1 June 1988 (1988-06-01), pages 49-53, XP011413639, ISSN: 0272-1708, DOI: 10.1109/37.477
- P.B.Nagy, W.S.Rosenberg, L.M. Stankovitis: "Non-invasive monitoring of acute intracranial mass lesions using ultrasonic fingerprinting", , 1998, pages 249-254, XP002745444, Retrieved from the Internet: URL:http://journals.cambridge.org/action/d isplayFulltext?type=1&fid=8061637&jid=OPL& volumeId=503&issueId=-1&aid=8061635&bodyId =&membershipNumber=&societyETOCSession= [retrieved on 2015-10-06]
- MOMENAN R ET AL: "IMAGE STAINING AND DIFFERENTIAL DIAGNOSIS OF ULTRASOUND SCANS BASED ON THE MAHALANOBIS DISTANCE", IEEE TRANSACTIONS ON MEDICAL IMAGING, IEEE SERVICE CENTER, PISCATAWAY, NJ, US, vol. 13, no. 1, 1 March 1994 (1994-03-01), pages 37-47, XP000440148, ISSN: 0278-0062, DOI: 10.1109/42.276143
- Michael F Insanal ET AL: "PATTERN RECOGNITION METHODS FOR OPTIMIZING MULTIVARIATE TISSUE SIGNATURES IN DIAGNOSTIC ULTRASOUND", IXASONIC IMAGING, 1 January 1986 (1986-01-01), pages 165-180, XP055217916, Retrieved from the Internet: URL:http://ac.els-cdn.com/0161734686900076 /1-s2.0-0161734686900076-main.pdf?_tid=d44 41f60-68e5-11e5-af6f-00000aacb35f&acdnat=1 443777382_7dfac1660de42203ef2b4eafa64d838b [retrieved on 2015-10-06]
- SCHMITZ G ET AL: "Tissue characterization of the prostate using Kohonen-maps", ULTRASONICS SYMPOSIUM, 1994. PROCEEDINGS., 1994 IEEE CANNES, FRANCE 1-4 NOV. 1994, NEW YORK, NY, USA,IEEE, US, 31 October 1994 (1994-10-31), page 1487, XP032084618, DOI: 10.1109/ULTSYM.1994.401872 ISBN: 978-0-7803-2012-3
- Huang Yu-Len ET AL: "-1 - Contour Detection for the Breast Tumor in Ultrasonic Images Using Watershed Segmentation *", , 1 January 2002 (2002-01-01), pages 1-20, XP055938616, Retrieved from the Internet: URL:http://dspace.fcu.edu.tw/bitstream/237 7/2248/1/ce07ics002002000318.PDF [retrieved on 2022-07-05]

## Description

### BACKGROUND:

### Technical Field

This disclosure relates to tissue characterization and more particularly to systems and methods for determining and employing subject-specific tissue signatures for characterizing similar tissues in the subject.

### Description of the Related Art

Tissue characterization has typically been used to try to identify or detect diseased tissue across a subject population, for example, as an aid to screening. There are many medical conditions, including primary or secondary cancer, where a patient may present with more than one pathological lesion of the same type or origin. Unfortunately, in many cases some of these lesions may be occult. In other words, the lesions are not identified by one or more imaging modalities and may remain undetected for some time. One example is in liver cancer, where by some reports up to 30% of patients may have additional cancerous lesions that are not identified until liver surgery has commenced, despite the patient having previously been imaged with multiple modalities. It is assumed that this occurs at least partly because no known imaging modality has 100% accuracy or sensitivity, for a number of reasons.

In the case of ultrasound, the appearance of pathological lesions may be subtle and easily confused with benign lesions or difficult to distinguish from surrounding tissue. Methods have been developed to try to improve the discrimination of pathological lesions, such as tissue characterization where certain imaging parameters, associated with pathology, are identified by previously examining a larger population of normal and pathological samples. However, to date these methods have gained limited acceptance because of the typically large variability in the characterization parameter values across a population and due to significant overlap seen between normal and pathological tissue. Both of these reduce the ability of tissue characterization to reliably identify pathology without presenting an unacceptable level of false positives.

Document US 2004/0064050 discloses a system and method for distinguishing between normal and potentially abnormal tissue. The system includes computer components for: generating and receiving ultrasonic waves; storing a tissue model; and analyzing received ultrasonic waves in the context of the tissue model.

Document Momenan R et al: "Characterization of tissue from ultrasound images" discloses a procedure for classifying tissue types from unlabelled acoustic measurements using unsupervised analysis. Unsupervised learning techniques are applied to discriminate between tissue types of two neighbouring organs.

Document P.B.Nagy et al: "Non-invasive monitoring of acute intracranial mass lesions using ultrasonic fingerprinting" discloses a continuous computer controlled monitoring system based on an ultrasound fingerprinting method. An ultrasonic detector directed at the general area of interest can be used to record and update the personal signature of a patient, which is then used as an ultrasonic fingerprint. Any abrupt change in signature indicates the need for further investigation.

Document US2007/036402 discloses a system for determining tissue abnormalities within at least one medical image from a first modality and at least one medical image from a second modality.

### SUMMARY

The invention is defined by the claims.

In accordance with the present principles, a system for identifying tissue is defined in claim 1.

A method for identifying tissue is defined in claim 9.

These and other objects, features and advantages of the present disclosure will become apparent from the following detailed description of illustrative embodiments thereof, which is to be read in connection with the accompanying drawings.

### BRIEF DESCRIPTION OF DRAWINGS

This disclosure will present in detail the following description of preferred embodiments with reference to the following figures wherein:
FIG. 1 is a block/flow diagram showing a system for identifying tissue in accordance with one embodiment;
FIG. 2 is a diagram showing different lesions in a patient to be identified in accordance with the present principles; and
FIG. 3 is a flow diagram showing a method for identifying tissue in accordance with an illustrative embodiment.

### DETAILED DESCRIPTION OF EMBODIMENTS

In accordance with the present principles, improved methods and systems are provided to identify lesions of a same type within a subject and hence better determine the true extent of disease, especially when using an imaging modality with limited accuracy or sensitivity (e.g., ultrasound). One approach described here uses some concepts of image-based tissue characterization, but overcomes many of the limitations of existing methods by using the patient as their own "control", thus significantly reducing the variability of the image-based characterization parameters and improving the likelihood for identifying pathological tissue using an imaging method with otherwise limited accuracy and sensitivity. This may be done by obtaining definitive confirmation of pathology for a particular suspicious lesion, such as from a biopsy or the like but may be from a first imaging modality with higher diagnostic accuracy, and then extracting values of the imaging parameters for the second (less accurate) imaging modality that are associated with that pathology-confirmed lesion. These extracted values for the second imaging modality are assumed then to be characteristic of that pathology, i.e., they represent a template or "tissue signature" for the pathology. All imaging planes for the second imaging modality obtained from the subject are searched for all instances of lesions or tissue regions whose characteristics "match" those of the "tissue signature".

Ultrasound is one example of an imaging modality that may in some circumstances have limited accuracy and sensitivity for detecting pathological lesions, especially when the lesions are small or the patient is technically difficult. In particularly useful embodiments, a lesion is identified, e.g., as being malignant, using a definitive method, such as biopsy or a definitive imaging method. Then, an ultrasound image of the same lesion is obtained to define an ultrasound "lesion signature", which characterizes the lesion according to its image properties (e.g., speckle stats, texture, etc.) or properties of the raw received echo data. A search is performed for "matching" tissue so that all similar malignant lesions can be automatically identified. This has applications in screening, diagnosis, staging and intervention, to name a few.

It should be understood that the present invention will be described in terms of detecting and diagnosing a lesion; however, the teachings of the present invention are much broader and are applicable to any procedure for characterizing tissue. In some embodiments, the present principles are employed in tracking or analyzing biological tissue. In particular, the present principles are applicable to internal tracking procedures or analysis of biological systems in all areas of the body such as the lungs, gastro-intestinal tract, excretory organs, blood vessels, etc. The elements depicted in the FIGS. may be implemented in various combinations of hardware and software and provide functions which may be combined in a single element or multiple elements.

The functions of the various elements shown in the FIGS. can be provided through the use of dedicated hardware as well as hardware capable of executing software in association with appropriate software. When provided by a processor, the functions can be provided by a single dedicated processor, by a single shared processor, or by a plurality of individual processors, some of which can be shared. Moreover, explicit use of the term "processor" or "controller" should not be construed to refer exclusively to hardware capable of executing software, and can implicitly include, without limitation, digital signal processor ("DSP") hardware, read-only memory ("ROM") for storing software, random access memory ("RAM"), non-volatile storage, etc.

Moreover, all statements herein reciting principles, aspects, and embodiments of the invention, as well as specific examples thereof, are intended to encompass both structural and functional equivalents thereof. Additionally, it is intended that such equivalents include both currently known equivalents as well as equivalents developed in the future (i.e., any elements developed that perform the same function, regardless of structure). Thus, for example, it will be appreciated by those skilled in the art that the block diagrams presented herein represent conceptual views of illustrative system components and/or circuitry embodying the principles of the invention. Similarly, it will be appreciated that any flow charts, flow diagrams and the like represent various processes which may be substantially represented in computer readable storage media and so executed by a computer or processor, whether or not such computer or processor is explicitly shown.

Furthermore, embodiments of the present invention can take the form of a computer program product accessible from a computer-usable or computer-readable storage medium providing program code for use by or in connection with a computer or any instruction execution system. For the purposes of this description, a computer-usable or computer readable storage medium can be any apparatus that may include, store, communicate, propagate, or transport the program for use by or in connection with the instruction execution system, apparatus, or device. The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette, a random access memory (RAM), a read-only memory (ROM), a rigid magnetic disk and an optical disk. Current examples of optical disks include compact disk - read only memory (CD-ROM), compact disk - read/write (CD-R/W), Blu-Ray^{™} and DVD

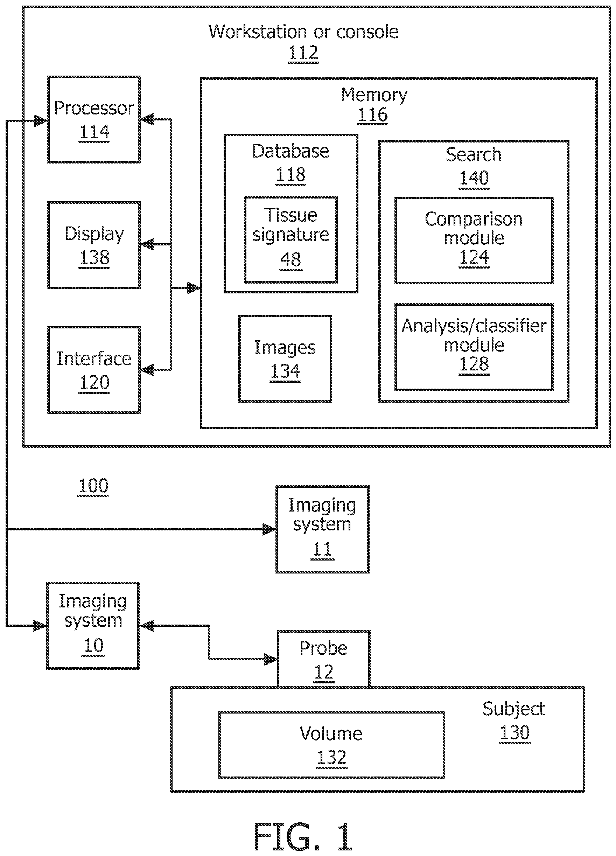

Referring now to the drawings in which like numerals represent the same or similar elements and initially to FIG. 1, a system for identifying tissue 100 is shown in accordance with one illustrative embodiment. System 100 may include a workstation or console 112 from which a procedure is supervised and/or managed. Workstation 112 preferably includes one or more processors 114 and memory 116 for storing programs and applications. Memory 116 may include a database 118 (or other memory structure) configured to store at least one tissue signature 48 for a target tissue 132 in an individual subject 130. It should be noted that the tissue signature and lesion signature may be used interchangeable throughout this disclosure.

The tissue signature 48 may be derived using one or more of the following: a first imaging modality 11, a second imaging modality 10, a biopsy or other tissue sample, etc. The first imaging modality 11 (and the second or additional imaging modalities, e.g., imaging system 10) are configured to collect image data or raw radio frequency (RF) data of the target 132. In some cases, the imaging system 11 can also be employed to obtain a definitive diagnosis of a particular lesion (the "reference lesion"), such as a cancer lesion. A location in tissue at which to determine the tissue signature 48 is derived by the definitive diagnosis. The definitive diagnosis may be obtained through biopsy, surgical excision followed by histopathology, etc. In some cases, an imaging modality with higher accuracy (e.g., contrast magnetic resonance (MR), computed tomography (CT), positron emission tomography (PET), contrast ultrasound (CEUS), etc.) may be employed to confirm the diagnosis of the tissue or lesion in the target 132. This lesion will be referred to as a reference lesion. Although a reference lesion is referred to, the reference may include any tissue.

Next, images from a given region, from several regions, from a bounded area, to all sections of the body may be obtained including the reference lesion and anywhere similar lesions might possibly be found. For example, all parts of both breasts may be imaged if the lesion includes, e.g., breast cancer. These images may be obtained before, after or as part of the procedure and may be acquired using a different imaging modality, such as ultrasound (e.g., imaging modality 10).

Next, an ultrasound pathology tissue signature is created for the reference lesion by defining values or a range of values for acoustic, morphological or textural image parameters associated with the lesion. Such parameters may include known parameters, including acoustic parameters (e.g., shadowing, speed of sound, attenuation), morphological parameters (e.g., lesion shape, border irregularity, etc.), and textural parameters such as first and second order speckle statistics (e.g., mean, median, skewness, kurtosis) or fractal dimension. Alternatively, the tissue signature could be created by analyzing the spectral characteristics and statistical moments of radiofrequency (RF) data from the lesion, such as mean frequency, frequency dependence of attenuation, or integrated backscatter. It is advantageous to understand the pathology of the reference lesion very well to further understand its typical features and characteristics.

The tissue signature 48 is stored in the database 118 or other memory storage structure. In one embodiment, the signature 48 includes identification of the boundaries of the lesion, so that only tissue within the lesion boundary is used to develop the pathology tissue signature. If the original diagnosis was made using a non-ultrasound imaging modality (e.g., imaging system 11), this step may benefit from registration of the ultrasound image (or volume) to the other modality image (or volume), so that the reference lesion can be more easily characterized and its margins defined by the non-ultrasound modality. The tissue signature itself may not be derived from the biopsy or histopathology. The biopsy or histopathology may instead be used to definitively indicate the location wherein the tissue signature should be generated.

A search module 140 automatically searches images 134 that have been collected and stored. The search module 140 includes a comparison module 124 that compares tissues, lesions, etc. with the tissue signature 48 and looks for tissues and/or lesions that have image parameters that match, within a defined range, the values defined as the pathology tissue signature 48. An image and tissue analysis module 128 classifies the tissues and/or lesions found by the search module 140, using the comparison module 124, as probably exhibiting the same pathology as the reference lesion 48. The analysis module 128 can assign a probability or match score based on the criteria of the signature 48 that a particular tissue or lesion matched the signature 48. In assigning a score the analysis module may consider in range/out of range values, image matching, size considerations, patient history, external statistics, etc. The probability score and how it relates to the ranges of the characterization parameters can be developed through clinical evaluations across multiple patients with normal and pathological lesions. Other factors that would be needed in the analysis may include the expected level of sensitivity and specificity to find matching lesions.

It should be understood that the tissue signature 48 is collected and derived from the patient directly and employed as a representative tissue signature for that patient. In other words, the patient's own tissue is employed as a sample for comparison with all other tissues in that patient. The patient is the control. The tissues compared may include a same organ, a particular region or a whole body scan. The tissue signature 48 can be stored and compared to future lesions or be compiled with other tissue signatures for that patient.

In one embodiment, a plurality of tissue signatures 48 is stored in the database 118. The plurality of tissue signatures 48 may include tissue signatures 48 taken at different times from the patient. The tissue signatures 48 may each be employed in a current procedure to perform a match and to compare the tissue signatures to current tissues in question. Each tissue signature 48 may then be scored against the tissues in question (or vice versa) to provide a higher confidence score for the tissues in question or to search for different lesion types within the patient. In one illustrative example, a current procedure identifies a malignant lesion (signature A) and a benign lesion (signature B). The searching module 140 may be employed to search out and identify both signature A and signature B lesions in the patient. In another embodiment, a lesion with signature C may have been obtained during a first procedure, and after time had passed a second procedure obtains a lesion with signature D. These two signatures C and D may be searched and/or employed for comparison to identify either or both signature tissue types during the current search. Other searches and tissue combinations are also contemplated.

Workstation 112 preferably includes a display 138 for viewing internal images of a subject (patient) 130 or volume 132. Display 138 may also permit a user to interact with the workstation 112 and its components and functions, or any other element within the system 100. This is further facilitated by an interface 120, which may include a keyboard, mouse, a joystick, a haptic device, or any other peripheral or control to permit user feedback from and interaction with the workstation 112.

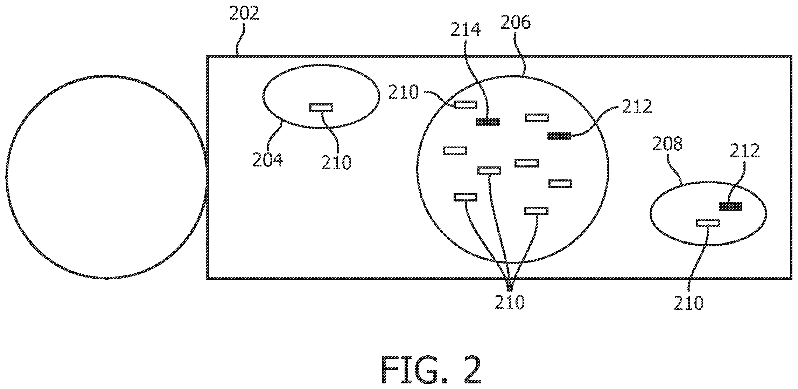

Referring to FIG. 2, one illustrative embodiment scans a patient 202 with ultrasound (e.g., 3D, although 2D may be employed), prior to an interventional therapeutic procedure and identifies a lesion or lesions 214 confirmed to be cancer, either by biopsy or by another more definitive imaging modality, such as contrast CT or contrast MRI. The known cancerous lesion 214 is then segmented from the ultrasound image, either through manual, automated or semi-automated methods which may be known in the art. Alternatively, the lesion 214 may be segmented on CT or MR, and this segmentation transferred to the ultrasound image by prior registration of the ultrasound image to the CT or MR image (methods to do the registration are also known). The images from different imaging modalities may be position tracked to make registration easier. This would ensure, e.g., that when searching, with ultrasound, for lesions that are similar to the reference or signature lesion, the signature lesion can be excluded since it is in a known location in tracked space.

Then, analysis of ultrasound morphology (e.g., shape, shadowing, etc.) and texture properties, such as speckle statistics (e.g., speckle mean, median, variance), fractal dimension, border irregularity, etc., is used to characterize the lesion or lesions 214 for that patient and thus to make a "template" or "signature" for that lesion 214.

Characterization methods may include known methods and methods developed in accordance with the present principles. During the procedure, the patient 202 is scanned again with a position-tracked ultrasound transducer, preferably in 3D, and the system (e.g., system 100 in FIG. 1) automatically searches for matches to the lesion signature. In this way, occult lesions can be identified before the procedure commences. In the present example, the position-tracked imaging scans some or all of the patient 202, e.g., organs 204, 206 and 208. The scanned organ 206 may be the same organ that includes the tissue signature or other organs or regions, e.g., 204, 208 that may include lesions, may be suspected of including lesions or may be scanned to discover lesions. In the example of FIG. 2, the scan uncovers two additional lesions 212 that match the tissue signature criteria, and other lesions or tissues 210 that do not match the tissue signature lesions. By having the patient's own tissue act as a control, greater accuracy in identifying questionable tissues is achieved.

The present principles may be employed in diagnosing or finding cancer, other lesions or growths, or similar pathologies within a patient. In particularly useful embodiments, the present principles are applicable to liver cancer, breast cancer, etc. However, there are many other potential applications for the present approach. One example is in the management of thyroid cancer. One of the challenges of correctly diagnosing, staging and following thyroid cancer is that a thyroid may contain many abnormal nodules (up to hundreds), only some of which may actually be cancer. Although a biopsy provides the most reliable initial diagnosis of cancer (excision and histopathology are usually required to fully characterize the cancer), it is often impractical (and unacceptable by the patient) to biopsy all of the nodules. Using the present principles, the clinician could instead biopsy the most suspicious nodules and, if a cancer is confirmed, then only biopsy nodules whose image characteristics match those of the cancerous nodule. This improves the chances of finding all of the cancer, and reduces the negative biopsy rate, patient discomfort and the time needed to perform the procedure.

Other applications may include first identifying a tissue or lesion that has a known pathology followed by an automatic search for similar lesions by the system. The search module 140 may return only the lesions that meet threshold criteria. This is applicable to many clinical applications in which it is difficult or time consuming to distinguish pathological lesions from benign lesions. Regarding applicable imaging modalities, while ultrasound is described, it should be understood that any imaging modality and especially ones that obtain volume data, since the method is much more likely to be successful for lesions that can be characterized in 3D, may be employed. For example, it may be challenging to identify all lesions in a CT or MR study, so if one lesion is confirmed to be pathology, all others could then be found quickly and automatically.

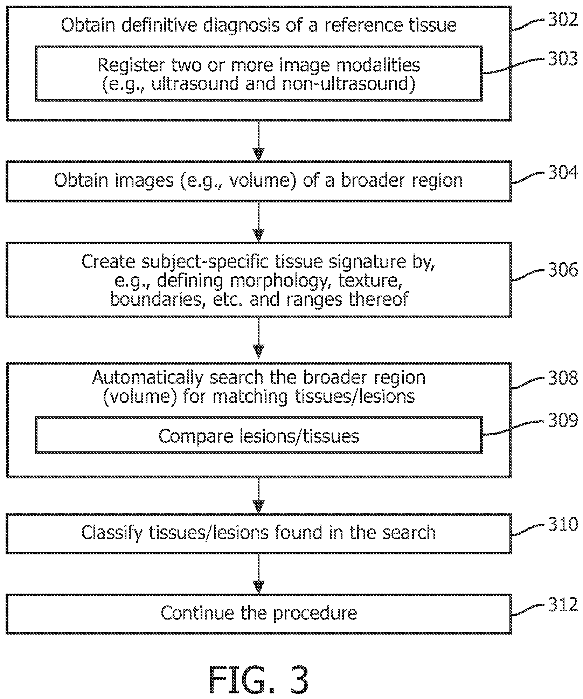

Referring to FIG. 3, a flow diagram shows a method for characterizing pathology using a lesion signature for a specific subject in accordance with illustrative embodiments.

In block 302, a definitive diagnosis of a particular lesion is obtained. The diagnosis may be obtained through a number of methods, e.g., biopsy, surgical excision followed by histopathology, a definitive imaging modality (e.g., contrast MR, CT, PET, etc.) or using other techniques. When a specific diagnosis is obtained the lesion is a reference lesion for that diagnosis. This may include creating an ultrasound pathology tissue signature for the reference tissue by defining values or a range of values for acoustic parameters, morphological image parameters associated with the reference tissue, textural image parameters associated with the reference tissue, spectral characteristics of radiofrequency data associated with a lesion and/or identification of boundaries of a lesion, so that only tissue within a lesion boundary is used to develop the tissue signature. In block 303, if a non-ultrasound imaging modality is employed, an ultrasound image or volume may be registered to a non-ultrasound image or volume provided by the non-ultrasound imaging modality such that the reference tissue is more easily characterized and its margins defined.

In block 304, images are obtained from a broader region, e.g., all sections of the body including the reference lesion and also anywhere similar lesions might possibly be found. For example, all parts of a same organ where the reference was determined. These images may be obtained before, during or after obtaining the reference lesion. The imaging of block 304 may be acquired using a different or the same imaging modality, such as ultrasound, as employed for block 302. It should be understood that the area of the scan may be user selected or determined based upon best practices or other criteria.

In block 306, an ultrasound pathology tissue signature is created for the reference lesion by defining values or a range of values for acoustic, morphological and/or textural image parameters associated with the lesion. This may include identification of the boundaries of the lesion, so that only tissue within the lesion boundary is used to develop the pathology tissue signature. If the original diagnosis was made using a non-ultrasound imaging modality, block 306 may benefit from registration of the ultrasound image (or volume) to the other modality image (or volume), so that the reference lesion can be more easily characterized and its margins defined.

In block 308, a search of the images from block 304 may be conducted automatically for tissues and/or lesions that have image parameters that match, within a defined range. The range values are those defined as the pathology tissue signature in block 306. In block 309, the tissue signature and tissues in the volume are compared to identify tissue matches in accordance with a score based upon the specified ranges of characteristics. In block 310, the tissues and/or lesions found in block 308 are classified as probably exhibiting the same pathology as the reference lesion. In other embodiments, multiple signatures may be employed during the search. In block 312, the procedure continues with further searching or creating a new signature.

In interpreting the appended claims, it should be understood that:
a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function; and
e) no specific sequence of acts is intended to be required unless specifically indicated.

Having described preferred embodiments for lesion signatures to characterize pathology for a specific subject (which are intended to be illustrative and not limiting), it is noted that modifications and variations can be made by persons skilled in the art in light of the above teachings. It is therefore to be understood that changes may be made in the particular embodiments of the disclosure disclosed which are within the scope of the the appended claims.

## Claims

1. A system for identifying tissue, comprising:
a subject-specific lesion signature (48) stored in a memory (116), the subject-specific lesion signature being derived from at least one ultrasound image, the lesion signature having specified ranges of characteristics based on analysis of a reference tissue of a subject having a confirmed pathology, wherein the pathology is confirmed by one or more of biopsy, histopathology or definitive imaging;
a searching module (140) stored in the memory and configured to search through images (134) of a volume of the subject to identify lesions in the images for comparison; and
a comparison module (124) configured to identify matches between the lesion signature of the reference tissue and lesions in the images of the volume, in accordance with a score based upon the specified ranges of characteristics.

2. The system as recited in claim 1, wherein the lesion signature (48) includes one or more values for: acoustic parameters, morphological parameters associated with a lesion, textural image parameters associated with a lesion, spectral characteristics of radiofrequency data associated with a lesion, and identification of boundaries of a lesion so that only tissue within a lesion boundary is employed to develop the lesion signature.

3. The system as recited in claim 1, wherein a location of the lesion signature (48) is determined by definitive diagnosis including one or more of biopsy, histopathology or definitive imaging.

4. The system as recited in claim 1, wherein the images (134) are obtained for the subject using ultrasound or include an ultrasound image registered thereto.

5. The system as recited in claim 1, wherein the lesion signature (48) includes a plurality of tissue signatures taken at different times from the same subject.

6. The system as recited in claim 1, wherein the lesion signature (48) includes a plurality of tissue signatures taken for different tissue types from the same subject.

7. The system as recited in claim 1, further comprising an image and tissue analysis module (128) configured to measure parameters and assigned ranges for defining the lesion signature.

8. The system as recited in claim 7, wherein the image and tissue analysis module (128) classifies tissues in accordance with respective scores.

9. A computer-implemented method for identifying tissue, comprising:
creating (306) for a pathology, which has been confirmed by one or more of biopsy, histopathology or definitive imaging, a subject-specific lesion signature derived from at least one ultrasound image, the lesion signature having specified ranges of characteristics based on analysis of a reference tissue of the subject having the confirmed pathology;
searching (308) through images of a volume of the subject to identify lesions in the images for comparison; and
comparing (309) between the lesion signature and lesions in the images of the volume to identify matches in accordance with a score based upon the specified ranges of characteristics.

10. The method as recited in claim 9, wherein the images (134) are obtained from a scan of the subject in one or more regions of the body.

11. The method as recited in claim 9, wherein creating (302) a subject-specific lesion signature includes creating an ultrasound pathology lesion signature for the reference tissue by defining values or a range of values for acoustic parameters, morphological image parameters associated with a lesion, textural image parameters associated with a lesion, spectral characteristics of radiofrequency data associated with a lesion, and identification of boundaries of a lesion, so that only tissue within a lesion boundary is used to develop the lesion signature.

12. The method as recited in claim 9, wherein if a non-ultrasound imaging modality is employed for confirming the pathology, the method further comprises:
registering (303) an ultrasound image or volume to a non-ultrasound image or volume provided by the non-ultrasound imaging modality such that the reference tissue is more easily characterized and its margins defined.

13. The method as recited in claim 9, further comprising classifying (310) tissues and/or lesions as exhibiting the same pathology as the reference tissue.

## Patentansprüche

1. System zum Identifizieren von Gewebe, umfassend:
eine subjektspezifische Läsionssignatur (48), die in einem Speicher (116) gespeichert ist, wobei die subjektspezifische Läsionssignatur von mindestens einem Ultraschallbild abgeleitet ist, wobei die Läsionssignatur spezifizierte Bereiche von Merkmalen aufweist, basierend auf einer Analyse eines Referenzgewebes eines Subjekts, das eine bestätigte Pathologie aufweist, wobei die Pathologie durch eine oder mehrere von Biopsie, Histopathologie oder definitiver Bildgebung bestätigt wird;
ein Suchmodul (140), das in dem Speicher gespeichert und konfiguriert ist, um Bilder (134) eines Volumens des Subjekts zu durchsuchen, um Läsionen in den Bildern zum Vergleich zu identifizieren; und ein Vergleichsmodul (124), das konfiguriert ist, um Übereinstimmungen zwischen der Läsionssignatur des Referenzgewebes und Läsionen in den Bildern des Volumens in Übereinstimmung mit einer Punktbewertung basierend auf spezifizierten Bereichen von Merkmalen zu identifizieren.

2. System nach Anspruch 1, wobei die Läsionssignatur (48) einen oder mehrere Werte für Folgendes beinhaltet:
akustische Parameter, morphologische Parameter, die mit einer Läsion assoziiert sind, Texturbildparameter, die mit einer Läsion assoziiert sind, spektrale Eigenschaften von Hochfrequenzdaten, die mit einer Läsion assoziiert sind, und Identifizierung von Grenzen einer Läsion, sodass nur Gewebe innerhalb einer Läsionsgrenze zur Entwicklung der Läsionssignatur verwendet wird.

3. System nach Anspruch 1, wobei eine Stelle der Läsionssignatur (48) durch eine definitive Diagnose bestimmt wird, die eine oder mehrere der folgenden Möglichkeiten beinhaltet: Biopsie, Histopathologie oder definitive Bildgebung.

4. System nach Anspruch 1, wobei die Bilder (134) für das Subjekt unter Verwendung von Ultraschall erlangt werden oder ein darauf registriertes Ultraschallbild beinhalten.

5. System nach Anspruch 1, wobei die Läsionssignatur (48) eine Vielzahl von Gewebesignaturen beinhaltet, die zu unterschiedlichen Zeitpunkten von demselben Subjekt genommen werden.

6. System nach Anspruch 1, wobei die Läsionssignatur (48) eine Vielzahl von Gewebesignaturen beinhaltet, die für verschiedene Gewebetypen von demselben Subjekt genommen werden.

7. System nach Anspruch 1, ferner umfassend ein Bild- und Gewebeanalysemodul (128), das konfiguriert ist, um Parameter und zugewiesene Bereiche zum Definieren der Läsionssignatur zu messen.

8. System nach Anspruch 7, wobei das Bild- und Gewebeanalysemodul (128) Gewebe gemäß den jeweiligen Punktbewertungen klassifiziert.

9. Computerimplementiertes Verfahren zum Identifizieren von Gewebe, umfassend:
Erzeugen (306) für eine Pathologie, die durch eine oder mehrere Biopsie(n), Histopathologie(n) oder definitive(n) Bildgebung(en) bestätigt wir (werden) einer subjektspezifischen Läsionssignatur, die aus mindestens einem Ultraschallbild abgeleitet ist, wobei die Läsionssignatur spezifizierte Bereiche von Merkmalen aufweist, die auf einer Analyse eines Referenzgewebes des Subjekts mit der bestätigten Pathologie basieren; Durchsuchen (308) von Bildern eines Volumens des Subjekts, um Läsionen in den Bildern zum Vergleich zu identifizieren; und
Vergleichen (309) zwischen der Läsionssignatur und Läsionen in den Bildern des Volumens, um Übereinstimmungen in Übereinstimmung mit einer Punktbewertung zu identifizieren, die auf den spezifizierten Bereichen von Merkmalen basiert.

10. Verfahren nach Anspruch 9, wobei die Bilder (134) aus einem Scan des Subjekts in einem oder mehreren Bereichen des Körpers erlangt werden.

11. Verfahren nach Anspruch 9, wobei ein Erzeugen (302) einer subjektspezifischen Läsionssignatur ein Erzeugen einer Ultraschall-Pathologie-Läsionssignatur für das Referenzgewebe durch Definieren von Werten oder eines Wertebereichs für akustische Parameter, morphologische Bildparameter, die mit einer Läsion assoziiert sind, texturale Bildparameter, die mit einer Läsion assoziiert sind, spektrale Charakteristika von Hochfrequenzdaten, die mit einer Läsion assoziiert sind, und Identifizieren von Grenzen einer Läsion beinhaltet, sodass nur Gewebe innerhalb einer Läsionsgrenze zum Entwickeln der Läsionssignatur verwendet wird.

12. Verfahren nach Anspruch 9, wobei, wenn eine Nicht-Ultraschall-Bildgebungsmodalität zum Bestätigen der Pathologie verwendet wird, das Verfahren ferner Folgendes umfasst:
Registrieren (303) eines Ultraschallbilds oder -volumens auf ein Nicht-Ultraschallbild oder -volumen, das von der Nicht-Ultraschall-Bildgebungsmodalität bereitgestellt wird, sodass das Referenzgewebe leichter charakterisiert und seine Ränder definiert werden können.

13. Verfahren nach Anspruch 9, ferner umfassend ein Klassifizieren (310) von Geweben und/oder Läsionen als solche, die dieselbe Pathologie aufweisen wie das Referenzgewebe.

## Revendications

1. Système pour identifier un tissu, comprenant:
une signature de lésion spécifique au sujet (48) stockée dans une mémoire (116), la signature de lésion spécifique au sujet étant dérivée d'au moins une image par ultrasons, la signature de lésion présentant des plages spécifiées de caractéristiques sur la base d'une analyse d'un tissu de référence d'un sujet présentant une pathologie confirmée, dans lequel la pathologie est confirmée par une ou plusieurs parmi une biopsie, une histopathologie ou une imagerie définitive;
un module de recherche (140) stocké dans la mémoire et configuré pour rechercher dans des images (134) d'un volume du sujet pour identifier des lésions dans les images à des fins de comparaison; et un module de comparaison (124) configuré pour identifier des correspondances entre la signature de lésion du tissu de référence et des lésions dans les images du volume, conformément à un score basé sur les plages spécifiées de caractéristiques.

2. Système selon la revendication 1, dans lequel la signature de lésion (48) inclut une ou plusieurs valeurs pour: des paramètres acoustiques, des paramètres morphologiques associés à une lésion, des paramètres d'image texturale associés à une lésion, des caractéristiques spectrales de données de radiofréquence associées à une lésion, et l'identification de limites d'une lésion de sorte que seul du tissu au sein d'une limite de lésion soit utilisé pour développer la signature de lésion.

3. Système selon la revendication 1, dans lequel un emplacement de la signature de lésion (48) est déterminé par un diagnostic définitif incluant un ou plusieurs parmi une biopsie, une histopathologie ou une imagerie définitive.

4. Système selon la revendication 1, dans lequel les images (134) sont obtenues pour le sujet en utilisant des ultrasons ou incluent une image par ultrasons enregistrée sur celles-ci.

5. Système selon la revendication 1, dans lequel la signature de lésion (48) inclut une pluralité de signatures de tissu prélevées à différents moments sur le même sujet.

6. Système selon la revendication 1, dans lequel la signature de lésion (48) inclut une pluralité de signatures de tissu prélevées pour différents types de tissus du même sujet.

7. Système selon la revendication 1, comprenant en outre un module d'analyse d'image et de tissu (128) configuré pour mesurer des paramètres et des plages assignées pour définir la signature de lésion.

8. Système selon la revendication 7, dans lequel le module d'analyse d'image et de tissu (128) classe les tissus selon des scores respectifs.

9. Procédé mis en œuvre par ordinateur pour identifier un tissu, consistant à:
créer (306) pour une pathologie, qui a été confirmée par une ou plusieurs parmi une biopsie, une histopathologie ou une imagerie définitive, une signature de lésion spécifique au sujet dérivée d'au moins une image par ultrasons, la signature de lésion présentant des plages spécifiées de caractéristiques sur la base de l'analyse d'un tissu de référence du sujet présentant la pathologie confirmée; rechercher (308) dans des images d'un volume du sujet pour identifier des lésions dans les images à des fins de comparaison; et comparer (309) entre la signature de lésion et les lésions dans les images du volume pour identifier des correspondances selon un score basé sur les plages spécifiées de caractéristiques.

10. Procédé selon la revendication 9, dans lequel les images (134) sont obtenues à partir d'un balayage du sujet dans une ou plusieurs régions du corps.

11. Procédé selon la revendication 9, dans lequel la création (302) d'une signature de lésion spécifique au sujet consiste à créer une signature de lésion de pathologie ultrasonore pour le tissu de référence en définissant des valeurs ou une plage de valeurs pour des paramètres acoustiques, des paramètres d'image morphologiques associés à une lésion, des paramètres d'image texturale associés à une lésion, des caractéristiques spectrales de données de radiofréquence associées à une lésion et l'identification de limites d'une lésion, de sorte que seul le tissu au sein d'une limite de lésion soit utilisé pour développer la signature de lésion.

12. Procédé selon la revendication 9, dans lequel si une modalité d'imagerie non ultrasonore est utilisée pour confirmer la pathologie, le procédé consiste en outre à: enregistrer (303) une image ou un volume ultrasonore sur une image ou un volume non ultrasonore fourni par la modalité d'imagerie non ultrasonore de sorte que le tissu de référence est plus facilement caractérisé et ses marges définies.

13. Procédé selon la revendication 9, consistant en outre à classifier (310) des tissus et/ou des lésions comme présentant la même pathologie que le tissu de référence.
